# NEW EUROPEAN PATENT SPECIFICATION

(11) **EP 1 438 960 B2**
(45) Date of publication and mention of the opposition decision: **19.12.2012**
(45) Mention of the grant of the patent: 21.03.2007
(21) Application number: 03290091.2
(22) Date of filing: 14.01.2003
(51) Int. Cl.: A61K 31/496, A61K 9/16, A61K 9/20

(54) **Compostion of itraconazole dispersed in a hydrophilic polymer having enhanced bioavailability**
Zusammensetzung in Form eines festen Dispersion enthaltend Itraconazol und ein hydrophilisches Polymer mit einer verbesserten Bioverfügbarkeit
Composition sous la forme d'une dispersion solide comprenant de l'itraconazole et un polymer hydrophilique dont la biodisponibilité est améliorée

(43) Date of publication of application: 21.07.2004
(73) Proprietor: Acino Pharma AG, 4253 Liesberg (CH)
(72) Inventor: Seth, Pawan, Irvine, CA 92612 (US)
(74) Representative: Lederer, Franz

(56) References cited:
- EP-A- 1 103 252
- WO-A-00/03697
- WO-A-98/42318
- WO-A1-01/41765
- WO-A1-98/31360
- FR-A- 2 803 748
- US-A- 5 633 015
- US-A1- 2002 176 894
- US-B1- 6 346 533

## Description

### BACKGROUND OF THE INVENTION

The invention relates to an itraconazole composition having enhanced dissolution and bioavailability, and to a process for preparing it.

Low-solubility pharmaceutical compounds often exhibit the drawback of insufficient dissolution in gastric fluids that prejudices obtaining a plasmatic concentration sufficient to achieve the therapeutical effects. Much work has been undertaken to achieve a sufficient plasmatic concentration necessary to reach the desired therapeutic effects.

Addition of a surfactant is a simple way to enhance dissolution. It allows to raise wettability of otherwise poorly-wettable drugs and hence to increase the dissolution rate of the drug. This technique, rather old, has been improved over the last years.

EP-A-0330532 provides co-micronization of a poorly-soluble drug (fenofibrate) with a surfactant (sodium laurylsulfate). The result is better than that obtained with pure mixing of the components.

WO-A-9831360 discloses fluidized bed granulation on an inert support of a suspension of a drug, where the suspension is in an aqueous solution comprising the hydrophilic polymer. Itraconazole is mentioned as a possible drug to which this invention may apply.

FR 2803 748 discloses a pharmaceutical oral preparation of itraconazole comprising itraconazole dispersed an hydrophilic polymer in a preferred weight ratio polymer:itraconazole of 3:1.

US 6,346,533 B1 discloses a method for preparing itraconazole in an amorphous form for improving bioavailability.

There is a need for an itraconazole composition that would exhibit an enhanced bioavailability. None of the above documents teaches or suggests the instant invention.

US 2002/0 176 894 discloses a itraconazol composition comprising a Vitamin E Polyethylene Glycol Succinate as emulsifier.

### SUMMARY OF THE INVENTION

The invention is defined by the claims.

According to one preferred embodiment, the weight ratio polymer:itraconazole is higher than or equal to 2.1. Said ratio may be from 1.9 to 5, especially from 2.1 to 4, preferably from 2.2 to 3.5.

### DETAILED DESCRIPTION

The invention is designed for itraconazole. The composition of the invention generally comprises the ingredients according to the following proportions: itraconazole: between 6 and 30wt%; polymer: between 11 and 65wt%; surfactant: between 0.1 and 3wt% and inert carrier: between 20 and 80wt%.

The inventor has found that, surprisingly, when the weight ration polymer:itraconazole is higher than 1.9, esp. higher than or equal to 2.1, then the bioavailability is greatly enhanced.

The inventor has found that surprisingly, when the granule as defined below is placed in an aqueous medium, dissolution is greatly improved compared to formulations manufactured using prior art techniques and that the high amount of polymer will dramatically improve bioavailability.

Also, and without wishing to be bound by theory, it has been observed that, surprisingly, itraconazole released from the granule in the aqueous medium shows a relatively small size, typically below 5µm, and even below such a value. This small size, combined with a rather higher amount of polymer allows for an enhanced dissolution.

Since itraconazole is available under a molecular state, and hydrophilic polymer is present in high amounts, these very small particles will easily go through the cell membrane, which allows obtaining a much better bioavailability, compared to the prior art.

In the framework of this invention, the expression "inert carrier" means any excipient, generally hydrophilic, pharmaceutically inert, crystalline or amorphous, in a particulate form, not leading to a chemical reaction under the operating conditions employed, and which is preferably soluble in an aqueous medium, notably in a gastric acid medium. Examples of such excipients are derivatives of sugars, such as lactose, saccharose, hydrolyzed starch (malto-dextrine), saccharose, mannitol, microcrystalline cellulose, etc. Preferably inert spheroids are used, such as those disclosed in "sugar spheres, US Pharmacopea XXV". These inert spheroids may be obtained by many processes, including extrusion of a water paste, followed by spheronization. These are available from Seppic, France or Werner, Germany. Mixtures are also suitable. The individual particle size of the inert hydrosoluble carrier can be, for example, between 50 and 500 microns, e.g. between 100 and 400 microns.

The expression "hydrophilic polymer" in the invention should be taken to mean any high molecular weight substance (greater, for example, than 300) having sufficient affinity towards water to dissolve therein and form a gel, allowing the final obtention of a film. Examples of such polymers are polyvinylpyrrolidone, polyvinylalcohol, hydroxypropylcellulose, hydroxymethylcellulose, hydroxypropylmethylcellulose, methylcellulose, ethylcellulose, gelatin, etc. or any other film-forming polymer known in the art (such as the polymers disclosed in "Handbook of Pharmaceutical excipients", 2nd Ed., 1994, American Pharmaceutical Association, Washington, ISBN 0 91730 66 8, by Wade A., Weller PJ.).Polymer blends are also suitable.

The preferred hydrophylic polymer is HPMC. The HPMC used in this invention is, for example, such that a 2% aqueous solution of the polymer at 20°C has a viscosity between 0,5 and 50cP, preferably between 2 and 25cP.

The term "surfactant" is used in its conventional sense in this invention. Any surfactant is suitable, whether it be amphoteric, non-ionic, cationic or anionic. Examples of such surfactants are: sodium lauryl sulfate, monooleate, monolaurate, monopalmitate, monostearate or another ester of (polyoxyethylene) sorbitane, sodium dioctylsulfosuccinate (DOSS), lecithin, stearylic alcohol, cetostearylic alcohol, cholesterol, polyoxyethylene ricin oil, polyoxyethylene fatty acid glycerides, poloxamer^{®} , etc. Mixtures of surfactants are also suitable. The preferred surfactant is the monostearate ester of (polyoxyethylene) sorbitane.

Additional agent, known in the art can be used together with the drug and the polymer, albeit this is not preferred.

The composition according to the invention is prepared by a novel process comprising spraying a solution of itraconazole and a hydrophilic polymer and a surfactant, onto the inert cores.

The method according to the invention consists in using the fluidized bed granulation principle, but with specific starting materials, in order to arrive at an improved dissolution profile and thus, at elevated bioavailability. In particular, the invention employs a solution of itraconazole in a solution of a hydrophylic polymer and a surfactant, where the solvent is an organic solvent. The solvent will solubilize both itraconazole and the polymer. This solvent can be a chlorinated solvent, such as methylene chloride (aka dichloromethane), or an alcane, such as hexane. The chlorinated solvent is preferred. It can be mixed with a co-solvent such as an alcohol, e.g. ethyl alcohol or isopropyl alcohol. The amount of the co-solvent can be up to 50% by volume, preferably up to 40%, especially between 25 and 40%.

The fluidized-bed granulation technique is widely used in the pharmaceutical industry for preparing capsules or tablets. Conventionally, according to the prior art, a powder or a mixture of powders (itraconazole + excipients) is put into suspension in the fluidized bed in a granulator, and a solution containing a binder and optionally, a surfactant, is sprayed onto this bed to form granules. The fluidized-bed granulation technique is well known to those skilled in the art and reference should be made to standard works such as for example "Die Tablette", by Ritschel, Ed. Cantor Aulendorf, pages 211-212.

The invention, as has been indicated, comprises spraying a solution in an organic solvent of itraconazole with a hydrophilic polymer and a surfactant onto an inert carrier. Following granulation, the granule formed consists of crystals of, for example, lactose, which are isolated (or possibly agglomerated together by the spray solution) and particles of itraconazole and polymer adhering to the crystal surface. The granule could similarly be constituted of coated crystals, which are agglomerated, or even of such an agglomerate having received a coating. The granule obtained with the instant process can be broadly defined as comprising inert carrier particles, which are either isolated or agglomerated together, and particles of itraconazole in admixture with a hydrophilic polymer and a surfactant adhering to the carrier particles surface.

The compositions according to the invention can also be prepared by other methods, for example by spraying a solution of itraconazole onto the hydrosoluble inert carrier and a surfactant, or simply by granulating the composition (e.g. on an oscillating screen having a proper mesh size).

The significant starting product is the solution of itraconazole. This solution is prepared by putting the itraconazole into solution in a solution comprising the hydrophylic polymer and a surfactant, in solution in an organic solvent. The surfactant is put into solution in the solvent (beaker + magnetic or vane stirrer). Next, the hydrophylic polymer (HPMC) is dispersed, while stirring, in the solution previously obtained. While still stirring, the itraconazole is dispersed in the form of a fine shower into the above solution, to form a solution. The order of these steps can be reversed. In case a co-solvent is used, part of the components can first be dissolved (or otherwise solubilized) in this co-solvent prior to its admixing with the solvent.

The itraconazole concentration in the solution is generally from 1 to 15% by weight, preferably from 2 to 10%. The hydrophylic polymer concentration in the solution is generally from 1 to 25% by weight, preferably 3 to 20%. The surfactant concentration in the solution is from 0.1 to 3% by weight, preferably below 2%. The total components (incl. but not limited to the three above) content in the solution is from 3 to 20% by weight, preferably 5 to 15%.

The invention also covers this novel solution.

Further studies have shown that is possible to specifically adapt the amount of solvent used, so as to avoid at the same time sticking problems due to too high concentration (more than 20%) and excessive spray rate due to too large volumes (more than 20g/minute/kg of carrier particles) .

The granules thus obtained can, if desired, be provided with an outer coating or compressed into tablets, or form agglomerates. The outer coating is applied using conventional coating techniques such as coating in a pan or fluidized bed coater.

The granule may be available for the patient in many forms, including in a hard capsule or in a tablet form. When administered in a capsule form, the granules will generally be mixed with a lubricant. When administered in a tablet form, the granules will generally be mixed with a disintegrant and a lubricant. The granules will generally represent 50 to 90% of the final weight of the pharmaceutical composition (tablet or capsule).

Examples of disintegrants are cross-linked polyvinylpyrrolidone, sodium croscarmellose, sodium carboxymethyl starch, modified starch or unmodified starch. Examples of lubricants are magnesium stearate, sodium stearyl fumarate, glycerol behenate and talc. Flow enhancing agents (such as colloidal silica) may also be used. The excipients can be any one traditionally used in the art. For more details about these excipients, one can refer to the disclosure in "Handbook of pharmaceutical excipients", American Pharmaceutical Association, 1994 ISBN 0 91730 66 8, by Wade A. and Weller P. When the granule obtained (whether subsequently coated or not) is compressed to form tablets, this step can be implemented using any conventional technique which is suitable, for example using alternating or rotating compressing equipment.

### EXAMPLES

The following example illustrates the invention without limiting it.

The following formulation is prepared.

| Compound | Amount (g) |
|---|---|
| Itraconazole | 100 |
| HPMC | 225 |
| Sorbitan monostearate | 5 |
| Sugar spheres 20-25 mesh | 180 |
| Methylene chloride | 1650 |
| Ethyl alcohol | 970 |

Surfactant is dissolved in alcohol under moderate stirring. HPMC then itraconazole are added to alcohol. Methylene chloride is then gradually added, resulting in solubilizing itraconazole and HPMC. The resulting solution is filtered on a mesh (100 mesh). It is then sprayed on the sugar spheres in fluidized bed granulator (Glatt^{®} GPCG1), equipped with a top injection system (Top Spray^{®}). The following parameters are used.

| | |
|---|---|
| Inlet Temperature(°C) | 45-48°C |
| Product Temperature (°C) | 40-45°C |
| Air Volume (m³/h) | 100-130 |
| Atomization Pressure (bar) | 2.5-3.0 |
| Nozzle diameter (mm) | 1.0 |
| Pump Speed (g/min) | 8-13 |

The resulting granules are filled in hard capsules (together with 15g of silica as filler). These capsules are then subjected to a dissolution test. These dissolution tests are carried out, in an apparatus according to the US Pharmacopea, XXIV, type II paddle, at 100 rpm, in a dissolution medium comprised of 1000 ml of HCl 0.1N.

The results (expressed in dissolved fraction in %) are the following.

| Time (min) | 0 | 15 | 30 | 45 | 60 | 90 |
|---|---|---|---|---|---|---|
| | 0 | 20 | 80 | 95 | 100 | 100 |

Also, the size of the itraconazole particle is measured (particles analyzer Nicomp^{®} 380, Particle sizing systems, Inc.). It is about 4 µm.

## Claims

1. A composition of itraconazole comprising an inert carrier covered with at least one layer containing said itraconazole in a micronized form having a size less then 5 µm, dispersed in a hydrophilic polymer and a surfactant in an amount between 0.1 and 3% by weight of the total composition, wherein the weight ratio polymer:itraconazole is equal to or higher than 1.9.

2. The composition of claim 1, wherein the weight ratio polymer:itraconazole is higher than or equal to 2.1.

3. The composition of claim 1 or 2, wherein the hydrophilic polymer is chosen in the group consisting in polyvinylpyrrolidone, polyvinylalcohol, hydroxypropylcellulose, hydroxymethylcellulose, hydroxypropylmethylcellulose, methylcellulose, ethylcellulose, gelatine and mixtures thereof.

4. The composition of claim 3, wherein the hydrophilic polymer is hydroxypropylmethylcellulose.

5. The composition of any one of claims 1 to 4, wherein the surfactant is present together with the hydrophilic polymer.

6. The composition of claim 5, wherein the surfactant is sodium lauryl sulfate; (polyoxyethylene) sorbitane monooleate, monolaureate, monopalmitate or monostearate; sodium dioctylsulfosuccinate, lecithin; stearylic alcohol; cetostearylic alcohol; cholesterol; polyoxyethylene ricin oil; polyoxyethylene fatty acid glyceride; poloxamer or a mixture thereof.

7. The composition of any one of claims 1 to 6, wherein the inert carrier is an inert sugar sphere.

8. The composition of claim 7, wherein the inert carrier has a particle size between 50 and 500 microns.

9. The composition of any one of claims 1 to 8, wherein itraconazole represents between 6 and 30 wt%.

10. The composition of any one of claims 1 to 9, wherein the polymer represents between 11 and 65 wt%.

11. The composition of any one of claims 1 to 10, wherein the surfactant represents between 0.1 and 3 wt%.

12. A granule composition comprising inert carrier particles, which are either isolated or agglomerated together, and particles of itraconazole with a particle size below 5 µm in admixture with hydroxpropylmethylcellulose, and a surfactant in an amount between 0.1 and 3% by weight of the total composition, adhering to the carrier particles surface, wherein the weight ratio polymer:itraconazole is equal to or higher than 1.9.

13. The composition of claim 12, wherein the weight ratio polymer:itraconazole is higher than or equal to 2.1.

14. The composition of any one of claims 1 to 13 in a capsule.

15. The composition of any one of claims 1 to 13 compressed into a tablet.

16. A process for preparing the composition of itraconazole of any one of claims 1 to 11 comprising an inert carrier covered with at least one layer containing itraconazole, dispersed in a hydrophilic polymer and a surfactant in an amount between 0.1 and 3% by weight of the total composition, wherein the weight ratio polymer:itraconazole is equal to or higher than 1.9, comprising the steps of:
(i) preparing a solution of itraconazole and polymer, and said surfactant, in an organic solvent; and
(ii) applying said solution onto an inert carrier.

17. The process of claim 16, wherein step (ii) comprises fluidized bed granulating.

18. The process of claim 16 or 17 wherein in step (i) the solvent is a chlorinated solvent, optionally in admixture with an alcohol.

19. The process of claim 18, wherein the solvent is methylene chloride, optionally in admixture with an alcohol.

20. The process of any one of claims 17 to 19 wherein the polymer is hydroxpropylmethylcellulose.

## Patentansprüche

1. Itraconazol-Zusammensetzung, die einen inerten Träger, der mit mindestens einer Schicht bedeckt ist, die das Itraconazol in mikronisierter Form mit einer Größe von weniger als 5 µm, dispergiert in einem hydrophilen Polymer und einem Tensid einer Menge zwischen 0,1 and 3 Gew.% der gesamten Zusammensetzung, enthält, wobei das Gewichtsverhältnis Polymer:Itraconazol gleich oder größer als 1,9 ist.

2. Zusammensetzung nach Anspruch 1, wobei das Gewichtsverhältnis Polymer:Itraconazol größer oder gleich 2,1 ist.

3. Zusammensetzung nach Anspruch 1 oder 2, wobei das hydrophile Polymer aus der Gruppe ausgewählt wird, die aus Polyvinylpyrrolidon, Polyvinylalkohol, Hydroxypropylcellulose, Hydroxymethylcellulose, Hydroxypropylmethylcellulose, Methylcellulose, Ethylcellulose, Gelatine und Gemischen davon besteht.

4. Zusammensetzung nach Anspruch 3, wobei das hydrophile Polymer Hydroxypropylmethylcellulose ist.

5. Zusammensetzung nach einem der Ansprüche 1 bis 4, wobei das Tensid gemeinsam mit dem hydrophilen Polymer vorhanden ist.

6. Zusammensetzung nach Anspruch 5, wobei das Tensid Natriumlaurylsulfat; Polyoxyethylen-Sorbitanmonooleat, -monolaurat, -monopalmitat oder -monostearat; Natriumdioctylsulfosuccinat, Lecithin; Stearylalkohol; Cetostearylalkohol; Cholesterin; Polyoxyethylen-Rizinusöl; Polyoxyethylen-Fettsäureglycerid; Poloxamer oder ein Gemisch davon ist.

7. Zusammensetzung nach einem der Ansprüche 1 bis 6, wobei der inerte Träger ein inertes Zuckerkügelchen ist.

8. Zusammensetzung nach Anspruch 7, wobei der inerte Träger eine Teilchengröße zwischen 50 und 500 Mikron aufweist.

9. Zusammensetzung nach einem der Ansprüche 1 bis 8, wobei das Itraconazol zwischen 6 und 30 Gew.% darstellt.

10. Zusammensetzung nach einem der Ansprüche 1 bis 9, wobei das Polymer zwischen 11 und 65 Gew.% darstellt.

11. Zusammensetzung nach einem der Ansprüche 1 bis 10, wobei das Tensid zwischen 0,1 und 3 Gew.% darstellt.

12. Granulatzusammensetzung, die inerte Trägerteilchen umfasst, welche entweder isoliert oder miteinander agglomeriert sind, und Itraconazolteilchen mit einer Teilchengröße unter 5 µm unter Beimischung von Hydroxypropylmethylcellulose sowie ein Tensid einer Menge zwischen 0,1 und 3 Gew.% der gesamten Zusammensetzung, die an der Oberfläche der Trägerteilchen haften, wobei das Gewichtsverhältnis Polymer:Itraconazol gleich oder größer als 1,9 ist.

13. Zusammensetzung nach Anspruch 12, wobei das Gewichtsverhältnis Polymer:Itraconazol größer oder gleich 2,1 ist.

14. Zusammensetzung nach einem der Ansprüche 1 bis 13 in einer Kapsel.

15. Zusammensetzung nach einem der Ansprüche 1 bis 13 zu einer Tablette komprimiert.

16. Verfahren zur Herstellung der Itraconazol-Zusammensetzung nach einem der Ansprüche 1 bis 11, die einen inerten Träger umfasst, der mit mindestens einer Schicht bedeckt ist, die Itraconazol, dispergiert in einem hydrophilen Polymer und einem Tensid einer Menge zwischen 0,1 und 3 Gew.% der gesamten Zusammensetzung, enthält, wobei das Gewichtsverhältnis Polymer:Itraconazol gleich oder größer als 1,9 ist, welches die Schritte umfasst:
(i) Herstellen einer Lösung von Itraconazol und Polymer sowie des Tensids in einem organischen Lösungsmittel; und
(ii) Auftragen dieser Lösung auf einen inerten Träger.

17. Verfahren nach Anspruch 16, wobei der Schritt (ii) Wirbelschicht-Sprühgranulation umfasst.

18. Verfahren nach Anspruch 16 oder 17, wobei in Schritt (i) das Lösemittel ein chloriertes Lösemittel, wahlweise unter Beimischung eines Alkohols ist.

19. Verfahren nach Anspruch 18, wobei das Lösemittel Methylenchlorid, wahlweise unter Beimischung eines Alkohols ist.

20. Verfahren nach einem der Ansprüche 17 bis 19, wobei das Polymer Hydroxpropylmethylcellulose ist.

## Revendications

1. Une composition d'itraconazole comprenant un support inerte recouvert d'au moins une couche contenant ledit itraconazole, sous une forme micronisée présentant une dimension inférieure à 5 µm, dispersé dans un polymère hydrophile et comprenant un agent tensioactif, en une proportion comprise entre 0,1 % et 3% en poids de ladite composition, dans laquelle le rapport pondéral polymère/itraconazole est égal ou supérieur à 1,9.

2. La composition selon la revendication 1, dans laquelle le rapport pondéral polymère/itraconazole est supérieur à ou égal à 2,1.

3. La composition selon la revendication 1 ou 2, dans laquelle le polymère hydrophile est choisi dans le groupe comprenant la polyvinylpyrrolidone, l'alcool polyvinylique, l'hydroxypropylcellulose, l'hydroxy-méthylcellulose, l'hydroxy-propylméthylcellulose, la méthylcellulose, l'éthyl-cellulose, la gélatine et leurs mélanges.

4. La composition selon la revendication 3, dans laquelle le polymère hydrophile est l'hydroxypropylméthylcellulose.

5. La composition selon une quelconque des revendications 1 à 4, dans laquelle l'agent tensioactif est présent en même temps que le polymère hydrophile.

6. La composition selon la revendication 5, dans laquelle l'agent tensioactif est du laurylsulfate de sodium, du (polyoxyéthylène) monooléate, monolaurate, monopalmitate ou monostéarate de sorbitan, du dioctylsulfosuccinate de sodium, de la lécithine, de l'alcool stéarylique, de l'alcool cétostéarylique, du cholestérol, de l'huile de ricin polyoxyéthylénée, du glycéride d'acide gras polyoxyéthyléné, un poloxamère ou leurs mélanges.

7. La composition selon une quelconque des revendications 1 à 6, dans laquelle le support inerte est une sphère de sucre inerte.

8. La composition selon la revendication 7, dans laquelle le support inerte présente une dimension particulaire comprise entre 50 et 500 microns.

9. La composition selon une quelconque des revendications 1 à 8, dans laquelle l'itraconazole représente entre 6 à 30% en poids de la composition.

10. La composition selon une quelconque des revendications 1 à 9, dans laquelle le polymère représente entre 11 à 65 % en poids de la composition.

11. La composition selon une quelconque des revendications 1 à 10, dans laquelle l'agent tensioactif représente entre 0,1 et 3 % en poids de la composition.

12. Une composition de granules comprenant des particules de support inertes, qui sont soit isolées soit agglomérées entre elles, comprenant des particules d'itraconazole présentant une dimension particulaire inférieure à 5 µm en mélange avec de l'hydroxypropylméthylcellulose, et comprenant un agent tensioactif en une proportion comprise entre 0,1 et 3% en poids du poids total de la composition, adhérant à la surface des particules support, composition dans laquelle le rapport pondéral polymère/itracouazole est égal ou supérieur à 1,9.

13. La composition selon la revendication 12, dans laquelle le rapport pondéral polymère/intraconazole est supérieur à ou égal à 2,1.

14. La composition selon une quelconque des revendications 1 à 13, sous forme de capsule.

15. La composition selon une quelconque des revendications 1 à 13, compressée sous forme de comprimé.

16. Un procédé de préparation d'une composition d'itraconazole selon une quelconque des revendications 1 à 11, comprenant un support inerte recouvert d'au moins une couche contenant ledit itraconazole, dispersé dans un polymère hydrophile et comprenant un agent tensioactif, en une proportion comprise entre 0,1 et 3 % en poids de ladite composition, dans laquelle le rapport pondéral polymère/itraconazole est égal ou supérieur à 1,9, comprenant les étapes de:
(i) préparation d'une solution d'itraconazole et d'un polymère, et dudit agent tensioactif dans un solvant organique, et
(ii) application de ladite solution sur un support inerte.

17. Le procédé selon la revendication 16, dans lequel l'étape (ii) comprend une granulation par lit fluidisé.

18. Le procédé selon la revendication 16 ou 17, dans lequel dans l'étape (i) le solvant est un solvant chloré, éventuellement mélangé à un alcool.

19. Le procédé selon la revendication 18, dans lequel le solvant est le chlorure de méthyle, éventuellement mélangé à un alcool.

20. Le procédé selon une quelconque des revendications 17 à 19, dans lequel le polymère est l'hydroxpropylméthylcellulose.
